# EUROPEAN PATENT APPLICATION

(11) **EP 4 130 845 A1**
(43) Date of publication of application: **08.02.2023**
(21) Application number: 21780769.2
(22) Date of filing: 29.01.2021
(51) Int. Cl.: G02B 23/24, A61B 1/00, A61B 1/12

(54) **OPTICAL DEVICE**

(30) Priority: 31.03.2020 JP 2020064617
(71) Applicant: FUJIFILM Corporation, Tokyo 106-8620 (JP)
(72) Inventor: TAKASAKI, Kosuke, ashigara-shi, Kanagawa 250-0193 (JP); SEKIZAWA, Yasuhiro, ashigara-shi, Kanagawa 250-0193 (JP)
(74) Representative: Klunker IP Patentanwälte PartG mbB
(86) International application number: PCT/JP2021/003279
(87) International publication number: WO 2021/199639

(57) **Abstract**

An optical device includes an optical window, a gas/liquid supply nozzle that includes an opening from which gas and liquid are jetted and selectively jets the gas and the liquid to wash an effective region of the optical window, a disposition surface on which the optical window and the nozzle are disposed, and a first hydrophobic portion that has a hydrophobic surface property and is formed in a part of a region of the disposition surface present between the effective region and the nozzle and between two tangent lines intersecting with a jet direction of the nozzle and extending to be tangent to the effective region from both ends of the opening in a width direction along the disposition surface.

## Description

### BACKGROUND OF THE INVENTION

### 1. Field of the Invention

A technique of the present disclosure related to an optical device.

### 2. Description of the Related Art

An endoscope is known as an example of an optical device including an optical window which light is incident on or emitted from. An observation window on which the light of a subject of which an image is to be picked up is incident is provided at a distal end of an insertion unit of an endoscope as an example of an optical window. A nozzle used to wash adhering materials, which adhere to the observation window, out is provided on a disposition surface where the observation window is disposed. The nozzle comprises an opening from which liquid, such as water, and gas, such as air used to blow water droplets off, is jetted. Liquid, such as water, and gas, such as air, are selectively jetted to the observation window from the nozzle, so that adhering materials can be removed from the observation window.

For example, JP2016-022006A discloses an endoscope in which an observation window protrudes to a predetermined height from a flat portion of a distal end cover provided at a distal end of an insertion unit and an inclined portion formed in a tapered shape toward the outside from a circular peripheral edge portion of the observation window is provided around the circular peripheral edge portion. At least a part of the surface of the inclined portion has a hydrophilic property.

In the endoscope disclosed in JP2016-022006A, water is jetted to the observation window from the nozzle first and air is then jetted to the observation window from the nozzle, so that water remaining on the surface of the observation window is removed. Since a hydrophilic region is provided around the observation window in the endoscope disclosed in JP2016-022006A, water remaining on the surface of the observation window is likely to move to the hydrophilic region provided around the observation window in a case where air is jetted from the nozzle. Accordingly, it is easy to remove liquid remaining on the observation window.

### SUMMARY OF THE INVENTION

However, there is a concern that water remains in the hydrophilic region, which is provided around the observation window, between the nozzle and the observation window in a case where water is jetted in the endoscope disclosed in JP2016-022006A. In a case where air is jetted after the jet of water in this case, water remaining in the hydrophilic region provided around the observation window is drawn into the flow of air and moved onto the surface of the observation window. For this reason, there is a problem that it is difficult to remove water from the surface of the observation window.

The technique of the present disclosure has been made in consideration of the above-mentioned circumstances, and an object of the present disclosure is to provide an optical device from which remaining water is easily removed as compared to the related art in a case where water remaining on an observation window is removed using the supply of air from a nozzle.

An optical device according to an aspect of the present disclosure comprises an optical window, a gas/liquid supply nozzle that includes an opening from which gas and liquid are jetted and selectively jets the gas and the liquid to wash an effective region of the optical window, a disposition surface on which the optical window and the nozzle are disposed, and a first hydrophobic portion that has a hydrophobic surface property and is formed in a part of a region of the disposition surface present between the effective region and the nozzle and between two tangent lines intersecting with a jet direction of the nozzle and extending to be tangent to the effective region from both ends of the opening in a width direction along the disposition surface.

It is preferable that a second hydrophobic portion is formed in a region that is adjacent to the effective region on a downstream side of the effective region in the jet direction of the nozzle.

It is preferable that a hydrophilic portion having a hydrophilic surface property is formed in a region outside the two tangent lines and adjacent to the first hydrophobic portion in the width direction.

It is preferable that the hydrophilic portion extends up to a downstream side of the effective region along the tangent lines.

It is preferable that at least the effective region of the optical window has a hydrophobic property.

It is preferable that the first hydrophobic portion is a first fine uneven structure in which a plurality of protruding portions are regularly arranged.

It is preferable that an interval between distal end surfaces of the protruding portions of the first fine uneven structure is 0.1 µm or more and 10 µm or less.

It is preferable that a height of the protruding portion of the first fine uneven structure is 2 µm or more and 50 µm or less.

It is preferable that the hydrophilic portion is a second fine uneven structure in which a plurality of protruding portions are randomly arranged.

It is preferable that a surface roughness of the second fine uneven structure is 0.5 µm or more and 1 µm or less.

According to the technique of the present disclosure, it is possible to provide an optical device from which remaining water is easily removed as compared to the related art in a case where water remaining on an observation window is removed using the supply of air from a nozzle.

### BRIEF DESCRIPTION OF THE DRAWINGS

Fig. 1 is a diagram showing the overall configuration of an endoscope apparatus.
Fig. 2 is a plan view of an endoscope as viewed from a distal end side.
Fig. 3 is a cross-sectional view of a distal end of an insertion unit of the endoscope and is a cross-sectional view taken along line A-A shown in Fig. 2.
Fig. 4 is a perspective view of a distal end part of the endoscope.
Fig. 5 is a perspective view of a first fine uneven structure.
Fig. 6 is a plan view of the first fine uneven structure as viewed from the distal end side of the endoscope.
Fig. 7 is a perspective view of a second fine uneven structure.
Figs. 8A, 8B, and 8C are diagrams illustrating the action of the endoscope during the supply of air and water, Fig. 8A shows an aspect in which water is jetted to an observation window from a nozzle, Fig. 8B shows an aspect in which the jet of water is stopped, and Fig. 8C shows an aspect in which air is jetted to the observation window from the nozzle.
Figs. 9A, 9B, and 9C are diagrams illustrating the action of an endoscope in the related art during the supply of air and water, Fig. 9A shows an aspect in which water is jetted to an observation window from a nozzle, Fig. 9B shows an aspect in which the jet of water is stopped, and Fig. 9C shows an aspect in which air is jetted to the observation window from the nozzle.
Fig. 10 is a plan view of the endoscope as viewed from a distal end side.
Fig. 11 is a plan view of the endoscope as viewed from the distal end side.
Fig. 12 is a cross-sectional view of a distal end of an insertion unit of the endoscope.
Fig. 13 is a perspective view of a distal end part of the endoscope.
Fig. 14 is a plan view of the endoscope as viewed from the distal end side.
Fig. 15 is a perspective view of a first fine uneven structure.
Fig. 16 is a plan view of the first fine uneven structure as viewed from the distal end side of the endoscope.
Fig. 17 is a perspective view of a first fine uneven structure.
Fig. 18 is a plan view of the first fine uneven structure as viewed from the distal end side of the endoscope.
Fig. 19 is a perspective view of a first fine uneven structure.
Fig. 20 is a plan view of the first fine uneven structure as viewed from the distal end side of the endoscope.
Fig. 21 is an external view of a surveillance camera.
Fig. 22 is a top view of a vehicle.
Fig. 23 is an external view of a headlight.
Fig. 24 is a plan view of a back camera as viewed from the rear side of a vehicle.

### DESCRIPTION OF THE PREFERRED EMBODIMENTS

### [First embodiment]

### [Configuration of endoscope apparatus]

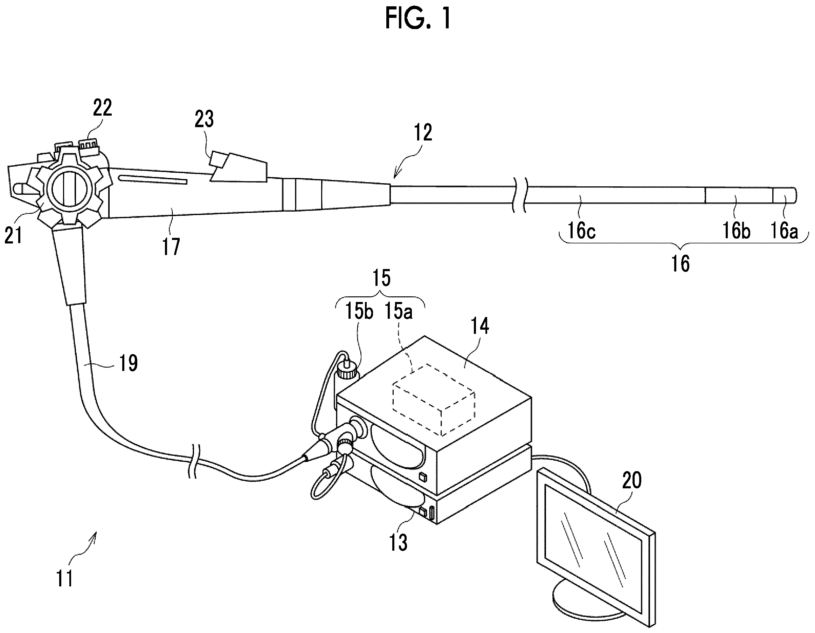

Fig. 1 is a diagram showing the overall configuration of an endoscope apparatus 11 comprising an endoscope according to a first embodiment of the present disclosure. As shown in Fig. 1, the endoscope apparatus 11 comprises an endoscope 12, a control device 13, a light source device 14, and an air/water supply device 15.

The endoscope 12 comprises an flexible insertion unit 16 that is to be inserted into an examinee's body, an operation unit 17 that is connected to a proximal end portion of the insertion unit 16 and is used for the grip of the endoscope 12 and the operation of the insertion unit 16, and a universal cord 19 that connects the operation unit 17 to the control device 13 and the light source device 14.

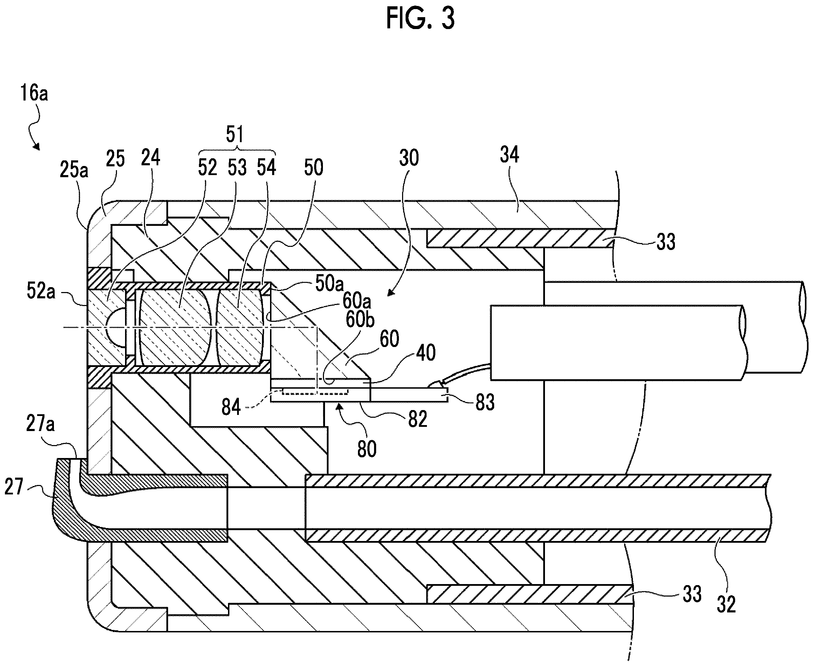

The insertion unit 16 comprises a distal end part 16a in which an image pickup unit 30 (see Fig. 3) is built, a bendable part 16b that is connected to a proximal end of the distal end part 16a and can be bent freely, and a flexible tube part 16c that is connected to a proximal end of the bendable part 16b and has flexibility. Here, the proximal end means one end, which is closer to the operation unit 17, of both ends of each member.

The operation unit 17 is provided with an operation member that includes angle knobs 21 used to bend vertically and laterally the bendable part 16b and air/water supply buttons 22 used to jet air and water from the distal end part 16a. Further, the operation unit 17 is provided with an inlet-side forceps port (not shown) through which a treatment tool, such as an electric scalpel, is inserted into a forceps channel inserted into the insertion unit 16.

The control device 13 is electrically connected to the light source device 14 and controls the operation of the endoscope apparatus 11 overall. The control device 13 supplies power to the endoscope 12 through a transmission cable, which is inserted into the universal cord 19 and the insertion unit 16, and controls the drive of an image pickup element 82 (see

Fig. 3). Further, the control device 13 acquires image pickup signals output from the image pickup element 82 through the transmission cable, and performs various types of image processing to generate image data. The image data generated by the control device 13 are displayed on a monitor 20, which is connected to the control device 13 through a cable, as an observation image.

The light source device 14 includes, for example, semiconductor light sources, such as light emitting diodes (LEDs), and the like, and supplies light to the distal end part 16a of the endoscope 12 through a light guide that is inserted into the universal cord 19 and the insertion unit 16.

The air/water supply device 15 includes a well-known air supply device (for example, a pump or the like) 15a that is built in the light source device 14 and supplies air and a washing water tank 15b that is provided outside the light source device 14 and stores washing water.

### <Configuration of distal end part of endoscope>

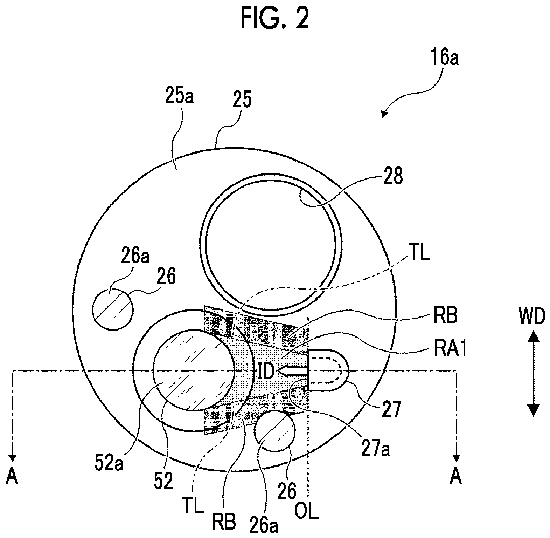

Fig. 2 is a plan view of the endoscope as viewed from a distal end side. Fig. 3 is a cross-sectional view of a distal end of the insertion unit of the endoscope and is a cross-sectional view taken along line A-A shown in Fig. 2.

As shown in Fig. 2, an observation window 52, two illumination windows 26, an air/water supply nozzle 27, and an outlet-side forceps port 28 are provided on a distal end surface of the distal end part 16a, that is, a distal end surface 25a of a distal end protection cap 25. The distal end protection cap 25 is made of, for example, a resin material, such as polysulfone.

The illumination windows 26 emit light, which is supplied from the light source device 14 and is guided by the light guide, from a light-emitting surface 26a toward an object to be observed present in a body cavity. The observation window 52 is a window through which light reflected from the object to be observed is taken into the distal end part 16a. The observation window 52 functions as a lens, which is closest to the distal end side, of an objective optical system 51 of the image pickup unit 30 in this example (see Fig. 3). The observation window 52 is an example of an optical window according to the technique of the present disclosure.

In this example, the entire region of a light incident surface 52a of the observation window 52 is an effective region. The effective region of the observation window 52 is at least a part of the region of the light incident surface 52a and refers to a region through which effective luminous flux contributing to image pickup passes. In more detail, light, which reaches an image pickup surface 84 (see Fig. 3) without vignetting in the middle, of light passing through the observation window 52 is effective luminous flux contributing to image pickup, and a region of the light incident surface 52a, which is defined by a position through which the maximum marginal ray of the effective luminous flux passes, is referred to as an effective region. In this example, all light passing through the light incident surface 52a is effective light contributing to image pickup. In this case, the entire region of the light incident surface 52a is the effective region of the observation window 52.

The two illumination windows 26 are disposed at symmetrical positions on both sides of the observation window 52. The air/water supply nozzle 27 is disposed at a position adjacent to the observation window 52 so that an opening 27a of the nozzle 27 faces the observation window 52. The nozzle 27 selectively jets air and water to the observation window 52 from the opening 27a according to the operation of the air/water supply button 22. The air/water supply nozzle 27 is an example of a gas/liquid supply nozzle according to the technique of the present disclosure.

As shown in Fig. 3, the distal end part 16a comprises a hard distal end part 24 and the distal end protection cap 25 that is mounted on the distal end side of the hard distal end part 24. The hard distal end part 24 is made of metal, such as stainless steel, and a plurality of through-holes are formed in the hard distal end part 24 in a longitudinal direction. Various components, such as the image pickup unit 30, an air/water supply channel 32, a forceps channel (not shown), and a light guide (not shown), are inserted into the respective through-holes of the hard distal end part 24. A rear end of the hard distal end part 24 is connected to distal ends of bendable pieces 33 of the bendable part 16b. Further, the outer periphery of the hard distal end part 24 is covered with an outer tube 34.

The image pickup unit 30 comprises an objective optical system 51 that forms an image on the image pickup surface 84 of the image pickup element 82 using light incident from the observation window 52, a lens barrel 50 that holds the objective optical system 51, a prism 60 that is connected to a proximal end 50a of the lens barrel 50, a cover glass 40 is adhered to the prism 60, and an image pickup element substrate 80 that is disposed to face a light-emitting surface 60b of the prism 60.

The objective optical system 51 comprises the observation window 52 functioning as a lens, a lens 53, and a lens 54. The lens barrel 50 holds the observation window 52, the lens 53, and the lens 54 therein.

A part of the image pickup element substrate 80 is provided with the image pickup element 82 having the image pickup surface 84, and the cover glass 40 is provided on the image pickup surface 84 of the image pickup element 82. A circuit unit 83 is provided at the rear end of the image pickup element substrate 80. The circuit unit 83 has a function to process image pickup signals output from the image pickup element 82, a function to transmit the image pickup signals to the control device 13, and the like.

The air/water supply nozzle 27 is connected to the air/water supply device 15 through the air/water supply channel 32.

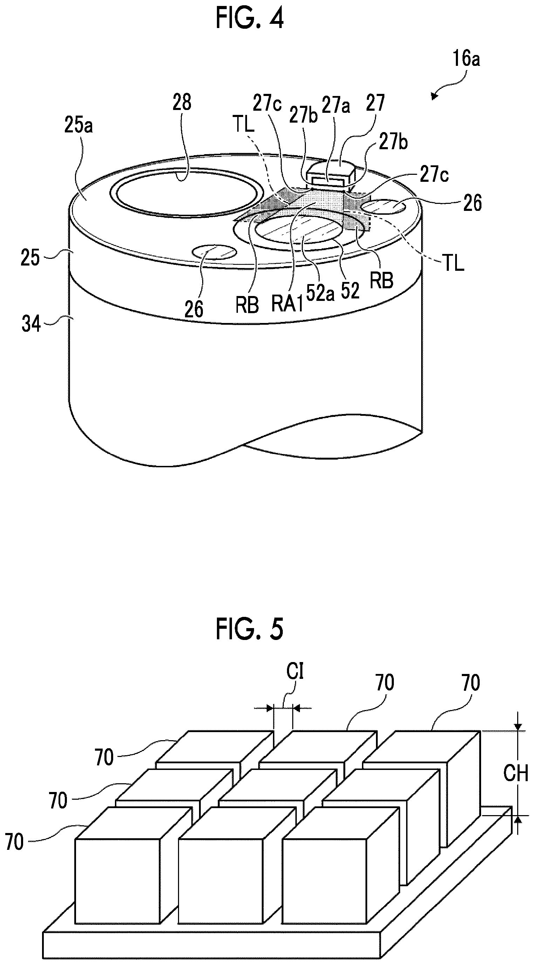

As shown in Figs. 2 and 4, the observation window 52 and the nozzle 27 are disposed on the distal end surface 25a of the distal end protection cap 25. The distal end surface 25a is an example of a disposition surface according to the technique of the present disclosure.

The distal end surface 25a comprises a first hydrophobic portion RA1 having a hydrophobic surface property. The first hydrophobic portion RA1 is formed in a part of the region of the distal end surface 25a present between the observation window 52 and the nozzle 27. More specifically, the region in which the first hydrophobic portion RA1 is formed is the entire region between two tangent lines TL that intersect with a jet direction ID of the nozzle 27 and extend to be tangent to the light incident surface 52a of the observation window 52 from both ends of the opening 27a of the nozzle 27 in a width direction WD along the distal end surface 25a. In this example, the entire region of the light incident surface 52a is an effective region according to the technique of the present disclosure as described above. In this example, the two tangent lines TL are tangent to the outer edge of the light incident surface 52a.

Here, water and air are jetted over a wide range from the opening 27a of the nozzle 27 in the width direction WD, but the jet direction ID refers to the direction of the central axis of a range to which water and air are to be jetted from the opening 27a. Further, "hydrophobic property" in the technique of the present disclosure means a property in which a static contact angle with respect to pure water is 110° or more.

Furthermore, as shown in Fig. 4, one end points of the two tangent lines TL are intersections 27c between end portions 27b of the opening 27a in the width direction WD and the distal end surface 25a. Further, the two tangent lines TL extend toward the light incident surface 52a of the observation window 52 from the respective intersections 27c. The other end points of the two tangent lines TL are contact points at which the two tangent lines are tangent to the outer edge of the light incident surface 52a. As shown in Figs. 2 and 4 by thin hatching, the region in which the first hydrophobic portion RA1 is formed is a region that is defined by the two tangent lines TL, a part of the outer edge of the light incident surface 52a, and a side of the opening 27a extending in the width direction WD.

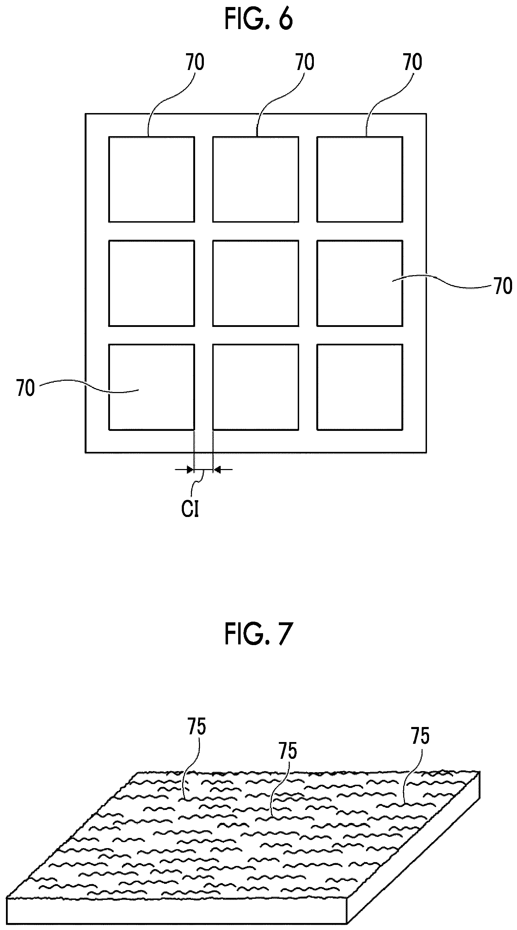

The first hydrophobic portion RA1 is formed of a first fine uneven structure in which a plurality of protruding portions are regularly arranged. For example, the first fine uneven structure is a structure in which a plurality of protruding portions 70 having the shape of a square column and substantially the same height are arranged at regular intervals in a grid pattern as shown in Figs. 5 and 6. An interval CI between the distal end surfaces of the protruding portions 70 of the first fine uneven structure is 0.1 µm or more and 10 µm or less. Further, a height CH of the protruding portion 70 is 2 µm or more and 50 µm or less in the first fine uneven structure. Machining for cutting grooves between the protruding portions 70 is performed on the first hydrophobic portion RA1 of the distal end surface 25a using an ultrashort pulse laser having a pulse width of femtoseconds to several picoseconds, so that the first fine uneven structure is formed.

Further, as shown in Figs. 2 and 4 by dark hatching, hydrophilic portions RB having a hydrophilic surface property are formed in regions outside the two tangent lines TL and adjacent to the first hydrophobic portion RA1 in the width direction WD. "Hydrophilic property" in the technique of the present disclosure means a property in which a static contact angle with respect to pure water is 15° or less.

As shown in Fig. 2, the regions in which the hydrophilic portions RB are formed are regions from one end to the other end of the tangent line TL in the jet direction ID and are region defined by lines parallel to the tangent lines TL in the width direction WD. It is preferable that the width of the hydrophilic portion RB in the width direction WD is a width allowing water, which flows into the hydrophilic portions RB from the first hydrophobic portion RA1, to be held not to flow back to the first hydrophobic portion RA1. Further, in this example, boundaries of the hydrophilic portions RB closer to a nozzle 27 are set on a line OL (see Fig. 2) extending in the width direction WD at an end edge of the observation window 52 of the nozzle 27.

As shown in Fig. 7, each of the hydrophilic portions RB is formed of a second fine uneven structure in which a plurality of protruding portions 75 having different heights are randomly arranged. The surface roughness of the second fine uneven structure is 0.5 µm or more and 1 µm or less. Cutting and melting are randomly performed on the entire region of the hydrophilic portions RB of the distal end surface 25a using an ultrashort pulse laser, so that the second fine uneven structure is formed.

Further, the effective region of the observation window 52 that is an example of an optical window has a hydrophobic property. In this example, since the entire region of the light incident surface 52a is an effective region, the entire region of the light incident surface 52a has a hydrophobic property. As with the first hydrophobic portion RA1, the light incident surface 52a of the observation window 52 is machined to have a hydrophobic property using an ultrashort pulse laser.

### [Action during supply of air and water]

An action in a case where the supply of air and water is performed to wash the light incident surface 52a of the observation window 52 in the endoscope 12 including the distal end part 16a having the above-mentioned configuration will be described.

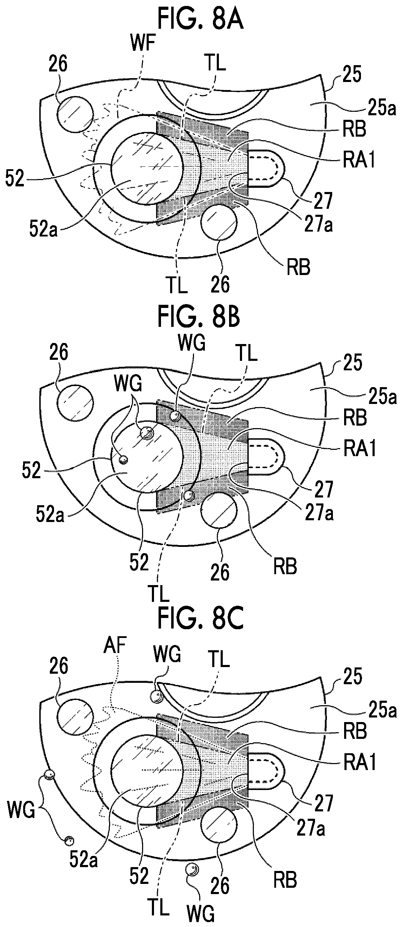

First, as shown in Fig. 8A, water WF is jetted to the light incident surface 52a of the observation window 52 from the opening 27a of the nozzle 27. In a case where water WF is jetted, the water WF passes through the first hydrophobic portion RA1 from the nozzle 27 and flows to the light incident surface 52a. Adhering materials adhering to the light incident surface 52a are washed out by the jetted water WF. In this case, not all the water WF jetted from the nozzle 27 reaches the light incident surface 52a and water droplets WG not reaching the light incident surface 52a are generated. The water droplets WG not reaching the light incident surface 52a tend to stay on the first hydrophobic portion RA1 formed in the region between the nozzle 27 and the light incident surface 52a. However, since the first hydrophobic portion RA1 has a hydrophobic surface property, it is difficult for the first hydrophobic portion RA1 to be wet by the water droplets WG and the first hydrophobic portion RA1 repels the water droplets WG For this reason, the water droplets WG flow to the outside without being staying on the first hydrophobic portion RA1 because of a jet force.

Accordingly, it is difficult for the water droplets WG to remain on the first hydrophobic portion RA1 as shown in Fig. 8B. Further, the hydrophilic portions RB are provided adjacent to the first hydrophobic portion RA1. Since the hydrophilic portions RB are likely to be wetted by the water droplets WG, the water droplets WG flowing to the outside from the first hydrophobic portion RA1 are likely to be guided to the hydrophilic portions RB. Furthermore, it is difficult for the water droplets WG guided to the hydrophilic portions RB to flow back to the first hydrophobic portion RA1, so that the water droplets WG remain on the hydrophilic portions RB as shown in Fig. 8B.

In a case where water WF is jetted to the light incident surface 52a, a part of the water WF remains on the light incident surface 52a as water droplets WG as shown in Fig. 8B. In order to remove such water droplets WG, air AF is jetted to the observation window 52 from the opening 27a of the nozzle 27 after the water WF is jetted.

In a case where air AF is jetted, the water droplets WG remaining on the light incident surface 52a of the observation window 52 are removed by the air AF as shown in Fig. 8C. As with the water WF, the air AF is jetted from the nozzle 27 passes through the first hydrophobic portion RA1 from the nozzle 27 and flows to the light incident surface 52a.

The first hydrophobic portion RA1 having a hydrophobic surface property is formed in the region between the nozzle 27 and the observation window 52. For this reason, since it is difficult for water droplets WG to remain on the first hydrophobic portion RA1 as shown in Fig. 8B after water WF is jetted, water droplets WG flowing to the light incident surface 52a from the first hydrophobic portion RA1 are caught in the air AF and reduced even in a case where air AF is jetted. Accordingly, in a case where water droplets WG remaining on the light incident surface 52a are removed by the jet of the air AF, the adhesion of other water droplets WG, which remain on the flow path of the air AF, to the light incident surface 52a is suppressed by the jetted air AF. Further, a part of water droplets WG remaining on the hydrophilic portions RB are blown to the outside of the hydrophilic portions RB by the air AF.

### [Effects]

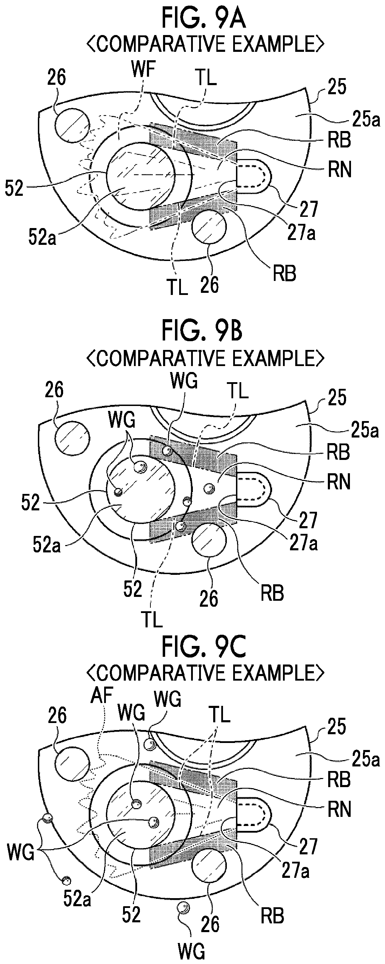

An example of an endoscope in the related art not provided with the first hydrophobic portion RA1 is shown in Figs. 9A, 9B, and 9C showing a comparative example. In a case where water WF is jetted to an observation window 52 from an opening 27a of a nozzle 27 in this case, the jetted water WF passes a region RN between the observation window 52 and the nozzle 27 as shown in Fig. 9A. In a case where the surface property of the region RN is not a hydrophobic property, the amount of a part of the jetted water WF remaining in the region RN as water droplets WG is large as shown in Fig. 9B.

In a case where air AF is jetted to the observation window 52 from the opening 27a of the nozzle 27 in this state, water droplets WG remaining on the light incident surface 52a are removed by the air AF but other water droplets WG remaining in the region RN positioned on the flow path of the air AF are moved onto the light incident surface 52a of the observation window 52 as shown in Fig. 9C. Then, other water droplets WG moved onto the light incident surface 52a remain on the light incident surface 52a. For this reason, there is a problem that it is difficult to remove water remaining on the light incident surface 52a of the observation window 52.

On the other hand, the endoscope 12 according to this embodiment comprises the first hydrophobic portion RA1 having a hydrophobic surface property as described above. The first hydrophobic portion RA1 is formed in a part of the region of the distal end surface 25a (an example of a disposition surface) present between the observation window 52 (an example of an optical window) and the nozzle 27 and between the two tangent lines TL that intersect with the jet direction ID of the nozzle 27 and extend to be tangent to the effective region from both ends of the opening 27a of the nozzle 27 in the width direction WD along the distal end surface 25a.

For this reason, even though water WF is jetted to the observation window 52 to wash the light incident surface 52a of the observation window 52, it is difficult for water droplets WG to remain on the first hydrophobic portion RA1 between the observation window 52 and the nozzle 27. After the supply of water for jetting water WF, the supply of air for jetting air AF is performed in order to remove water droplets WG remaining on the observation window 52. Even in a case where the supply of air is performed, the movement of other water droplets WG, which are caught in the air AF, onto the light incident surface 52a of the observation window 52 from the first hydrophobic portion RA1 present between the observation window 52 and the nozzle 27 is suppressed. Even in a case where the supply of air is performed after the supply of water as described above, the movement of other water droplets WG, which remain on the flow path of the air AF, to the observation window 52 is suppressed. Accordingly, it is easy to remove water droplets WG, which are an example of remaining liquid, from the light incident surface 52a of the observation window 52.

Further, the hydrophilic portions RB having a hydrophilic surface property are provided in the regions outside the two tangent lines TL and adjacent to the first hydrophobic portion RA1 in the width direction WD. Because of such a configuration, it is easy to guide water droplets WG to the hydrophilic portions RB from the first hydrophobic portion RA1 and it is difficult for water droplets WG to flow back to the first hydrophobic portion RA1 during the supply of water. For this reason, it is more difficult for water droplets WG to remain on the first hydrophobic portion RA1 positioned on the flow path of the air AF. As a result, since the adhesion of water droplets WG, which remain on the flow path of the air AF, to the observation window 52 is further suppressed during the supply of air, it is easier to remove water droplets WG from the light incident surface 52a of the observation window 52.

Furthermore, as shown in Fig. 2, the hydrophilic portions RB are formed in a region closer to the observation window 52 than the line OL that extends in the width direction WD of the opening 27a of the nozzle 27. In this way, the hydrophilic portions RB are formed only in the region closer to the observation window 52 than the line OL extending in the width direction WD of the opening 27a of the nozzle 27 and the hydrophilic portions RB are not formed in a region positioned on one side of the line OL where the nozzle 27 is positioned. Accordingly, the remaining of water droplets WG around the nozzle 27 is suppressed. For this reason, in a case where the supply of air is performed after the supply of water, the amount of water droplets WG present around the nozzle 27 caught in the jetted air AF is reduced. Therefore, the movement of the water droplets WG onto the light incident surface 52a of the observation window 52 is further suppressed. As a result, the amount of water droplets WG adhering to the light incident surface 52a of the observation window 52 after the supply of air is further reduced. Accordingly, it is easier to remove water droplets WG from the light incident surface 52a of the observation window 52.

Moreover, the light incident surface 52a of the observation window 52 has a hydrophobic property. Since the light incident surface 52a repels water droplets WG during the supply of air because of such a configuration, it is easy to remove water droplets WG from the light incident surface 52a.

Further, the first hydrophobic portion RA1 is formed of the first fine uneven structure in which a plurality of protruding portions are regularly arranged in this example. For example, ultrashort pulse laser machining is performed on the distal end surface 25a, so that this first fine uneven structure can be formed. For this reason, it is easy to reduce manufacturing costs including a material cost and a machining cost as compared to a case where coating using, for example, a fluorine-based coating agent or the like is performed.

Furthermore, the interval CI (see Fig. 5) between the protruding portions 70 of the first fine uneven structure is 0.1 µm or more and 10 µm or less in this example. Because of such a configuration, the first fine uneven structure can have an appropriate hydrophobic property. Specifically, machining in which the interval CI is smaller than 0.1 µm requires an expensive device, such as a device generating an electron beam, which causes a problem that the cost is too high. Further, in a case where the interval CI is larger than 10 µm, water is caused to enter recessed portions between the protruding portions 70 by Laplace force. Since the recessed portions are connected in a machined region, water spreads. For this reason, there is a problem that a hydrophobic property is not achieved. Accordingly, it is preferable that the interval CI between the protruding portions 70 is 0.1 µm or more and 10 µm or less in the first fine uneven structure.

Moreover, the height CH (see Fig. 5) of the protruding portion 70 of the first fine uneven structure is 2 µm or more and 50 µm or less in this example. Because of such a configuration, the first fine uneven structure can have an appropriate hydrophobic property. Specifically, in a case where the height CH is smaller than 2 µm, water is caused to enter between the protruding portions 70 (recessed portions in this example) by Laplace force. Since the recessed portions are connected in a machined region, water spreads. For this reason, there is a problem that a hydrophobic property is not achieved. Further, in a case where the height CH is 50 µm or more, a physical strength is insufficient, which causes a problem that the structure is broken during use. For this reason, it is preferable that the height CH (see Fig. 5) of the protruding portion 70 of the first fine uneven structure is 2 µm or more and 50 µm or less.

Furthermore, in this example, each of the hydrophilic portions RB is formed of a second fine uneven structure in which a plurality of protruding portions are randomly arranged. For example, ultrashort pulse laser machining is performed on the distal end surface 25a, so that this second fine uneven structure can be formed as with the first fine uneven structure. For this reason, it is easy to reduce manufacturing costs including a material cost and a machining cost as compared to a case where coating using, for example, a silica-based coating agent or the like is performed.

Further, the surface roughness of the second fine uneven structure is 0.5 µm or more and 1 µm or less in this example. Because of such a configuration, the second fine uneven structure can have an appropriate hydrophilic property. Specifically, since the surface area of the hydrophilic portions RB cannot be increased in a case where the surface roughness is lower than 0.5 µm, there is a problem that hydrophilicity in a desired level cannot be achieved. Furthermore, in a case where the surface roughness is higher than 1 µm, the hydrophilic portions RB get wet, and water spreads, the protruding portions having roughened surfaces serve as walls and water does not spread. For this reason, there is a problem that hydrophilicity in a desired level cannot be achieved. Accordingly, it is preferable that the surface roughness of the second fine uneven structure is 0.5 µm or more and 1 µm or less.

### [Second embodiment]

In an endoscope 12 according to a second embodiment, a second hydrophobic portion RA2 is added to the endoscope 12 according to the first embodiment in addition to a first hydrophobic portion RA1 and the range of hydrophilic portions RB is changed.

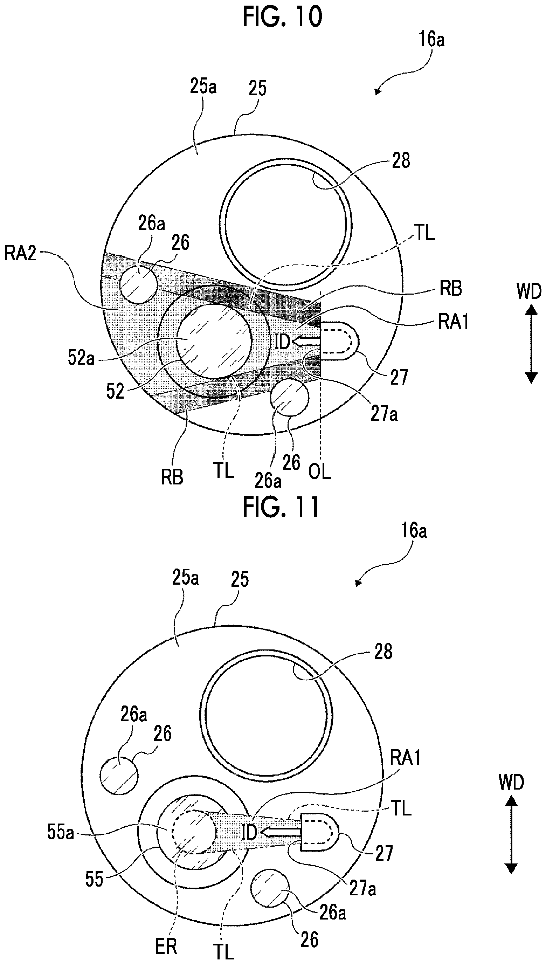

As shown in Fig. 10, the endoscope 12 according to the second embodiment comprises the same first hydrophobic portion RA1 as that of the first embodiment.

Further, in the endoscope 12 according to the second embodiment, the second hydrophobic portion RA2 is formed in a region that is adjacent to the light incident surface 52a on the downstream side of the light incident surface 52a in the jet direction ID of the nozzle 27.

Furthermore, the endoscope 12 comprises hydrophilic portions RB that have a hydrophilic surface property and are provided around the first hydrophobic portion RA1 to be adjacent to tangent lines TL. The hydrophilic portions RB extend up to the downstream side of the light incident surface 52a along the tangent lines TL. Boundaries of the hydrophilic portions RB closer to the nozzle 27 are set on a line OL extending in the width direction WD of the opening 27a of the nozzle 27 as in the first embodiment.

Since the second hydrophobic portion RA2 is formed in the region that is adjacent to the light incident surface 52a on the downstream side of the light incident surface 52a in the jet direction ID of the nozzle 27 as described above, water WF reaching the downstream side of the light incident surface 52a during the supply of water is repelled by the second hydrophobic portion RA2. Accordingly, it is difficult for water droplets WG to remain on the downstream side of the light incident surface 52a. For this reason, since it is difficult for water droplets WG to remain on the second hydrophobic portion RA2, the back flow of water droplets WG to the light incident surface 52a from the downstream side of the light incident surface 52a is suppressed. Therefore, it is easier to remove water droplets WG from the light incident surface 52a.

Further, since the hydrophilic portions RB are formed to extend up to the downstream side of the light incident surface 52a (an example of an effective region) along the tangent lines TL, it is easy to guide water droplets WG, which are repelled from the first hydrophobic portion RA1 and the second hydrophobic portion RA2, to the hydrophilic portions RB during the supply of water. Furthermore, it is difficult for the water droplets WG, which are guided to the hydrophilic portions RB, to flow back to the first hydrophobic portion RA1 and the second hydrophobic portion RA2. For this reason, since it is more difficult for water droplets WG to remain on the first hydrophobic portion RA1 and the second hydrophobic portion RA2 and the adhesion of water droplets WG to the light incident surface 52a is suppressed, it is easier to remove water droplets WG from the light incident surface 52a.

### [Modification examples]

In the technique of the present disclosure, the above-mentioned various embodiments and various modification examples can also be appropriately combined. Further, it goes without saying that the present disclosure is not limited to the respective embodiments and can employ various configurations without departing from a gist.

For example, the entire region of the light incident surface 52a of the observation window 52 is set as an effective region in the above-mentioned examples, but an effective region ER of a light incident surface 55a of an observation window 55 may be a part of the light incident surface 55a as shown in Fig. 11. The effective region ER of the observation window 55 refers to a region through which effective luminous flux contributing to image pickup passes as described above.

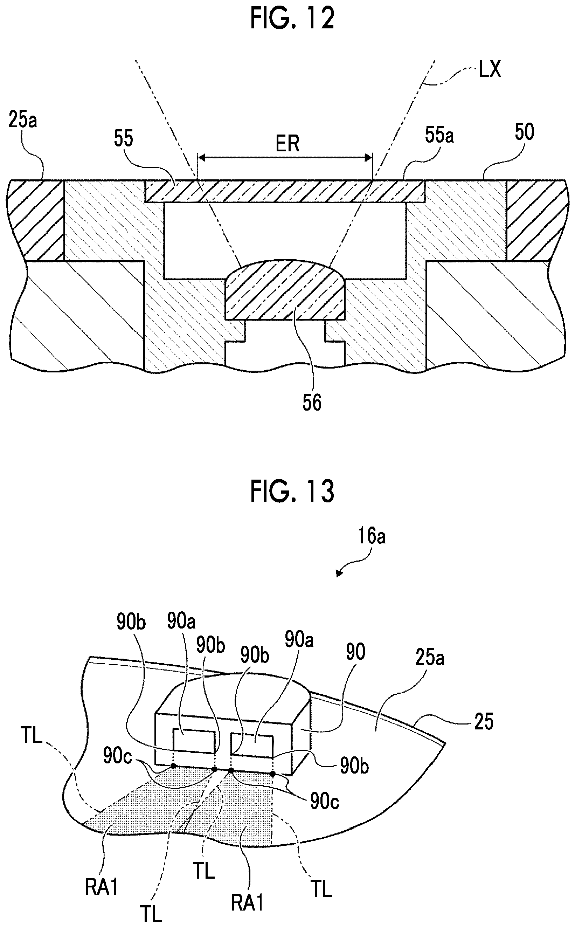

In Fig. 12, dotted lines LX indicate the maximum marginal ray of effective luminous flux contributing to image pickup, and an effective region ER is defined on the light incident surface 55a by a position through which the maximum marginal ray LX passes. The effective region ER may be a part of the light incident surface 55a. In a case where a part of the light incident surface 55a is the effective region ER as described above, one end of each of the tangent lines TL defining the first hydrophobic portion RA1 is positioned inside the outer edge of the light incident surface 55a as shown in Fig. 11.

In Fig. 12, the observation window 55 is a cover glass not having a lens function, and an objective optical system 56 is disposed inside the observation window 55. The observation window 52 shown in Fig. 3 has a lens function, but the observation window 55 may not have a lens function as shown in Fig. 12.

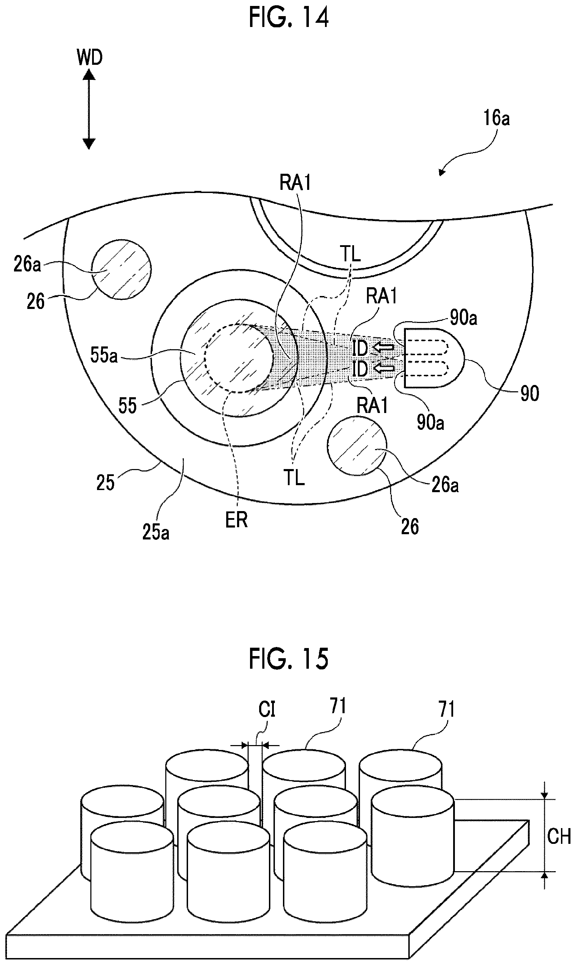

Further, one nozzle 90 may be provided with a plurality of openings 90a as shown in Figs. 13 and 14. An example in which one nozzle 90 includes two openings 90a is shown in Figs. 13 and 14 as an example. In a case where one nozzle 90 is provided with the plurality of openings 90a, a region between two tangent lines TL extending to be tangent to the effective region ER from both ends of each opening 90a may be set as the first hydrophobic portion RA1.

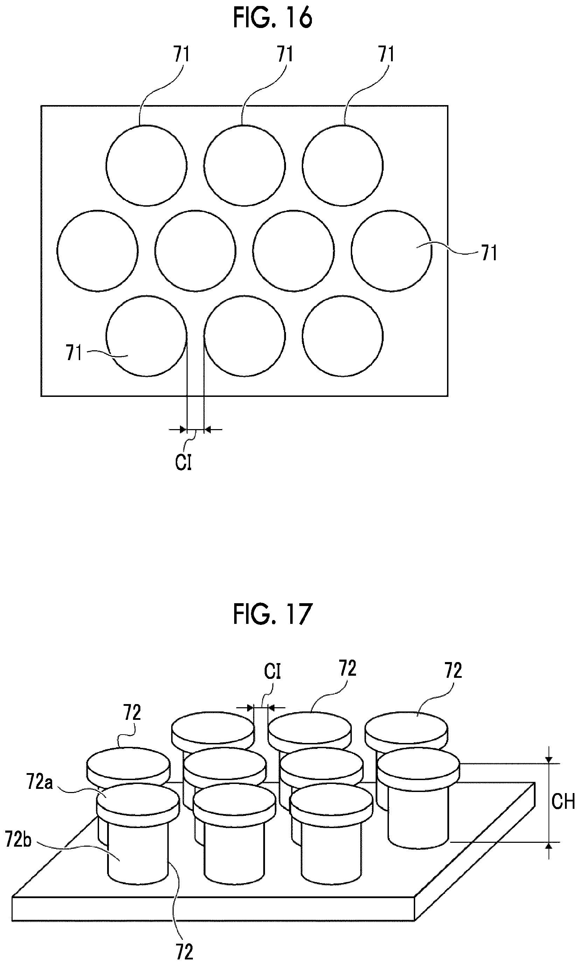

Furthermore, a first fine uneven structure of the first hydrophobic portion is not limited to an aspect in which the plurality of protruding portions 70 having the shape of a square column and the same height are arranged at regular intervals in a grid pattern as described above, and may be an aspect in which a plurality of protruding portions 71 having a columnar shape and the same height are arranged at regular intervals in zigzag as shown in Figs. 15 and 16.

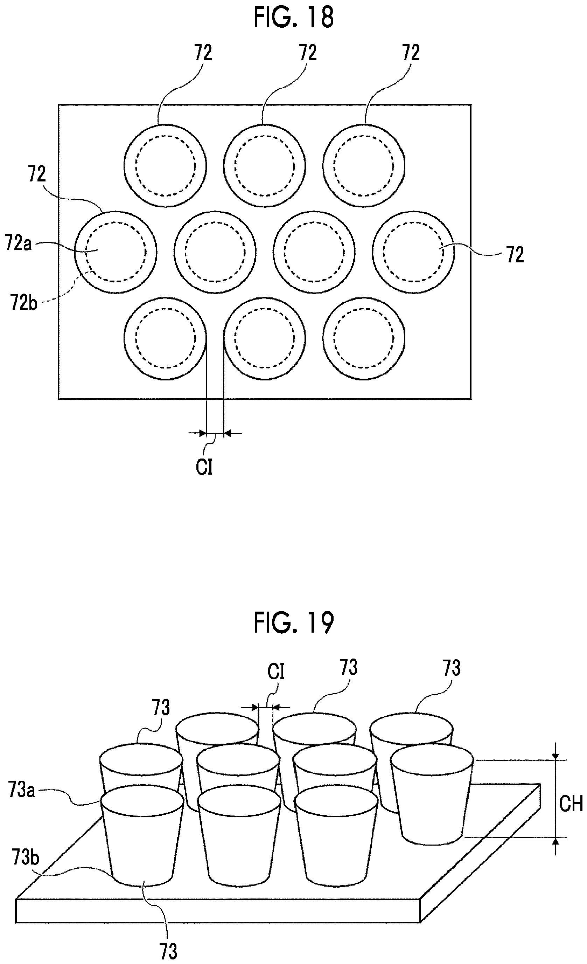

Moreover, as shown in Figs. 17 and 18, the diameter of a distal end portion 72a of a protruding portion 72 of the first fine uneven structure may be larger than the diameter of a columnar proximal end portion 72b thereof. In order to form the protruding portion in such a shape, for example, the columnar protruding portion 72 is formed using ultrashort pulse laser machining and the distal end portion thereof can be then melted and crushed to form the distal end portion 72a. The protruding portion 72 is formed in a columnar shape in the example shown in Figs. 17 and 18, but may be formed in the shape of a prism.

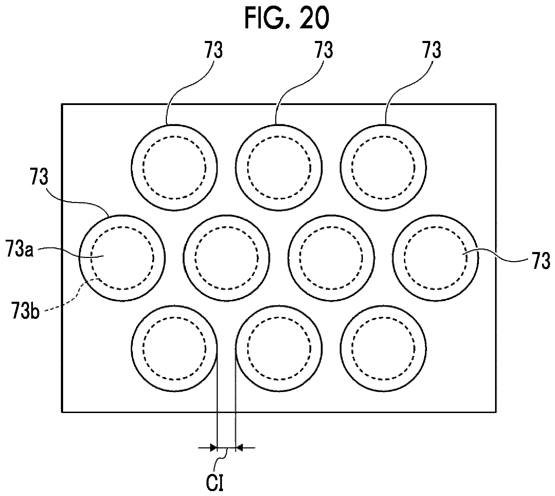

Further, as shown in Figs. 19 and 20, a protruding portion 73 of the first fine uneven structure may be formed in a conical shape in which a distal end 73a is thicker than a proximal end 73b. In order to form the protruding portion in such a shape, for example, an ultrashort pulse laser may be obliquely incident on a surface on which the protruding portions 73 are to be formed to perform machining. The protruding portion 73 is formed in a conical shape in the example shown in Figs. 19 and 20, but may be formed in a pyramid shape in which a distal end side is thicker than a proximal end side.

Further, the shape of the protruding portion of the first fine uneven structure can be various shapes, such as a pyramid shape in which a distal end side is thinner than a proximal end side or a conical shape in which a distal end side is thinner than a proximal end side, in addition to the above-mentioned shapes.

Furthermore, an aspect in which the plurality of protruding portions of the first fine uneven structure are arranged may be various aspects without being limited to an aspect in which the plurality of protruding portions are arranged at regular intervals in a grid pattern and an aspect in which the plurality of protruding portions are arranged at regular intervals in zigzag.

Moreover, the first hydrophobic portion RA1 and the second hydrophobic portion RA2 are not limited to an aspect that is realized in a case where the first fine uneven structure is formed on the distal end surface 25a of the distal end protection cap 25, and may be realized in a case where a region to serve as a hydrophobic portion is coated with a hydrophobic coating agent, such as a fluorine-based coating agent or a silicone-based coating agent. In a case where the region to serve as a hydrophobic portion is coated with a coating agent separate from the distal end protection cap 25 as described above, it is possible to select an appropriate material corresponding to performance required for the first hydrophobic portion RA1 and the second hydrophobic portion RA2.

Further, the hydrophilic portions RB are not limited to an aspect that is realized in a case where the second fine uneven structure is formed on the distal end surface 25a of the distal end protection cap 25, and may be realized in a case where a region to serve as a hydrophilic portion is coated with a hydrophilic coating agent, such as a silica-based coating agent. In a case where the region to serve as a hydrophilic portion is coated with a coating agent separate from the distal end protection cap 25 as described above, it is possible to select an appropriate material corresponding to performance required for the hydrophilic portions RB.

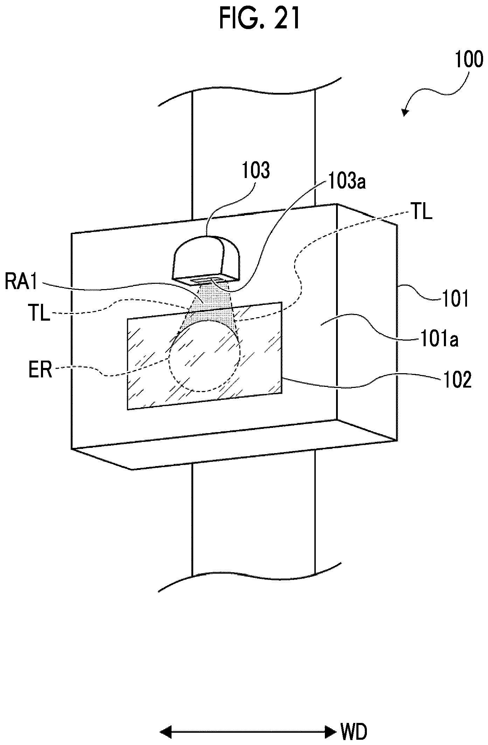

Furthermore, an optical device to which the technique of the present disclosure is applied is not limited to the endoscope and the technique of the present disclosure may be applied to a surveillance camera 100 as shown in Fig. 21. The surveillance camera 100 comprises an observation window 102 and a gas/liquid supply nozzle 103 on a front surface 101a (corresponding to a disposition surface) of a housing 101. A camera (not shown), which picks up an image, is disposed on a back surface of the observation window 102. A part of the region of the observation window 102 through which effective luminous flux contributing to image pickup passes is an effective region ER. A first hydrophobic portion RA1 having a hydrophobic surface property is formed in a region between the effective region ER and the nozzle 103 and between two tangent lines TL, which extend to be tangent to the effective region ER from both ends of an opening 103a in a width direction WD, on the front surface 101a (corresponding to a disposition surface) of the housing 101.

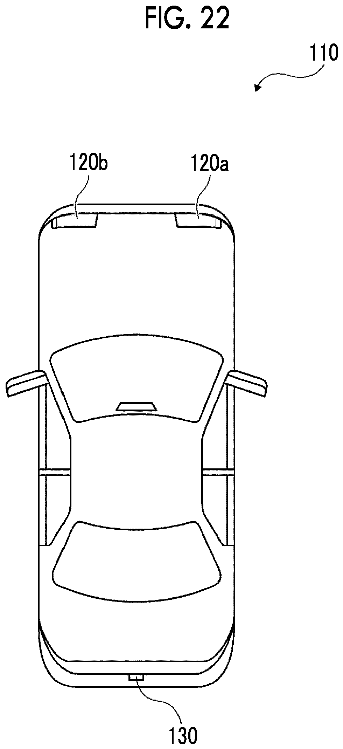

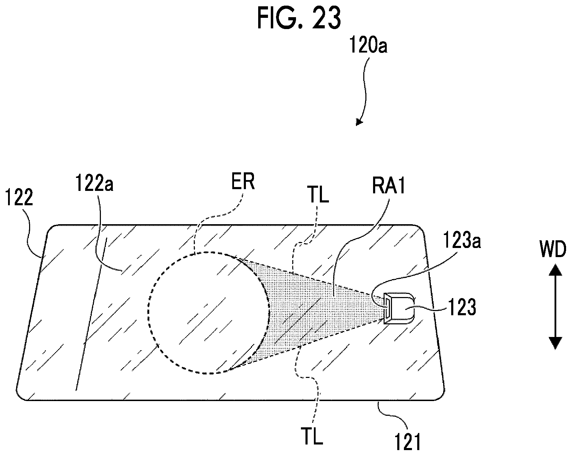

Further, the technique of the present disclosure may be applied to headlights 120a and 120b provided on the front side of a vehicle 110 as shown in Figs. 22 and 23. Fig. 23 shows the headlight 120a provided on the front right side of the vehicle 110. The headlight 120a comprises a transparent cover 122 on a front surface of a housing 121. A gas/liquid supply nozzle 123 is provided on a front surface 122a (corresponding to a disposition surface) of the transparent cover 122. An illumination light (not shown) is disposed on a back surface of the transparent cover 122. In a case where the technique of the present disclosure is applied to an optical device for illumination as described above, an optical window serves as an illumination window through which light is emitted from a light source. In the example shown in Fig. 23, the transparent cover 122 serves as an illumination window. A part of the region of the front surface 122a (corresponding to the disposition surface) of the transparent cover 122 through which effective luminous flux contributing to illumination passes is an effective region ER. A first hydrophobic portion RA1 having a hydrophobic surface property is formed in a region between the effective region ER and the nozzle 123 and between two tangent lines TL, which extend to be tangent to the effective region ER from both ends of an opening 123a in a width direction WD, on the front surface 122a (corresponding to the disposition surface) of the transparent cover 122. The headlight 120a provided on the front right side and the headlight 120b provided on the front left side have shapes symmetrical with each other, and includes the same components.

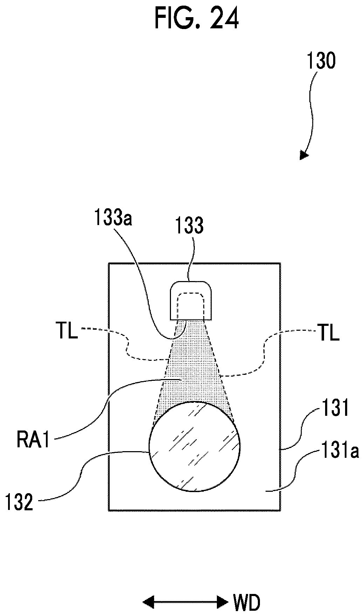

Furthermore, the technique of the present disclosure may be applied to a back camera 130 provided on the rear side of the vehicle 110 as shown in Figs. 22 and 24. The back camera 130 comprises an observation window 132 and a gas/liquid supply nozzle 133 on an image pickup side surface 131a (corresponding to a disposition surface) of a housing 131. A camera (not shown), which picks up an image, is disposed on a back surface of the observation window 132. In this example, the entire region of the observation window 132 is an effective region. A first hydrophobic portion RA1 having a hydrophobic surface property is formed in a region between the observation window 132 and the nozzle 133 and between two tangent lines TL, which extend to be tangent to the observation window 132 from both ends of an opening 133a in a width direction WD, on the image pickup side surface 131a (corresponding to a disposition surface) of the housing 131.

Further, an optical device to which the technique of the present disclosure is applied may be any optical device in addition to the above-mentioned optical devices as long as the optical device is an optical device comprising an optical window and a gas/liquid supply nozzle.

Furthermore, it is preferable that the first hydrophobic portion is a part of the region of a disposition surface present between an effective region of the optical window and the nozzle and is the entire region of a hydrophobization-recommended region between two tangent lines intersecting with the jet direction of the nozzle and extending to be tangent to the effective region from both ends of an opening in a width direction along the disposition surface. However, the first hydrophobic portion is not limited to such an aspect. In a case where 80% or more of the hydrophobization-recommended region is used as the first hydrophobic portion even if it is difficult for the entire region of a hydrophobization-recommended region to be used as the first hydrophobic portion because of restrictions in machining and the like, an effect of easily removing liquid remaining on the optical window can be obtained. Further, in a case where 90% or more of the hydrophobization-recommended region is used as the first hydrophobic portion, an effect of more easily removing liquid remaining on the optical window can be obtained as compared to a case where 80% or more of the hydrophobization-recommended region is used as the first hydrophobic portion.

Furthermore, the present disclosure is not limited to an aspect in which the first hydrophobic portion is completely adjacent to the hydrophilic portion, and an interval of 1 mm or less may be formed between the first hydrophobic portion and the hydrophilic portion. In a case where an interval of 1 mm or less is formed between the first hydrophobic portion and the hydrophilic portion even if it is difficult for the first hydrophobic portion and the hydrophilic portion to be completely adjacent to each other because of restrictions in machining and the like, it is possible to make water droplets be easily guided to the hydrophilic portions from the first hydrophobic portion and to make it difficult for the water droplets, which are guided to the hydrophilic portions, to flow back to the first hydrophobic portion during the supply of water.

The description contents and shown contents having been described above are the detailed description of portions according to the technique of the present disclosure, and are merely an example of the technique of the present disclosure. For example, the description of the configuration, functions, actions, and effects having been described above is the description of examples of the configuration, functions, actions, and effects of the portions according to the technique of the present disclosure. Accordingly, it goes without saying that unnecessary portions may be deleted or new elements may be added or replaced in the description contents and shown contents described above without departing from the scope of the technique of the present disclosure. Further, the description of common technical knowledge and the like, which allow the technique of the present disclosure to be embodied and do not need to be particularly described, is omitted in the description contents and shown contents, which have been described above, to avoid complication and to facilitate the understanding of portions according to the technique of the present disclosure.

All documents, patent applications, and technical standards disclosed in this specification are incorporated in this specification by reference such that the incorporation of each of the documents, the patent applications, and the technical standards by reference is specific and is as detailed as that in a case where the documents, the patent applications, and the technical standards are described individually.

The entire contents of the present disclosure of Japanese Patent Application No. 2020-064617, filed March 31, 2020, are incorporated in this specification by reference. All documents, patent applications, and technical standards disclosed in this specification are incorporated in this specification by reference so that the incorporation of each of the documents, the patent applications, and the technical standards by reference is specific and is as detailed as that in a case where the documents, the patent applications, and the technical standards are described individually.

## Claims

1. An optical device comprising:
an optical window;
a gas/liquid supply nozzle that includes an opening from which gas and liquid are jetted and selectively jets the gas and the liquid to wash an effective region of the optical window;
a disposition surface on which the optical window and the nozzle are disposed; and
a first hydrophobic portion that has a hydrophobic surface property and is formed in a part of a region of the disposition surface present between the effective region and the nozzle and between two tangent lines intersecting with a jet direction of the nozzle and extending to be tangent to the effective region from both ends of the opening in a width direction along the disposition surface.

2. The optical device according to claim 1,
wherein a second hydrophobic portion is formed in a region that is adjacent to the effective region on a downstream side of the effective region in the jet direction of the nozzle.

3. The optical device according to claim 1 or 2,
wherein a hydrophilic portion having a hydrophilic surface property is formed in a region outside the two tangent lines and adjacent to the first hydrophobic portion in the width direction.

4. The optical device according to claim 3,
wherein the hydrophilic portion extends up to a downstream side of the effective region along the tangent lines.

5. The optical device according to any one of claims 1 to 4,
wherein at least the effective region of the optical window has a hydrophobic property.

6. The optical device according to any one of claims 1 to 5,
wherein the first hydrophobic portion is a first fine uneven structure in which a plurality of protruding portions are regularly arranged.

7. The optical device according to claim 6,
wherein an interval between distal end surfaces of the protruding portions of the first fine uneven structure is 0.1 µm or more and 10 µm or less.

8. The optical device according to claim 6 or 7,
wherein a height of the protruding portion of the first fine uneven structure is 2 µm or more and 50 µm or less.

9. The optical device according to claim 3 or 4,
wherein the hydrophilic portion is a second fine uneven structure in which a plurality of protruding portions are randomly arranged.

10. The optical device according to claim 9,
wherein a surface roughness of the second fine uneven structure is 0.5 µm or more and 1 µm or less.
